# EUROPÄISCHE PATENTSCHRIFT

(11) **EP 0 772 586 B1**
(45) Veröffentlichungstag und Bekanntmachung des Hinweises auf die Patenterteilung: **09.12.1998**
(21) Anmeldenummer: 95932734.7
(22) Anmeldetag: 13.09.1995
(51) Int. Cl.: C07C 227/06, C07C 229/24

(54) **VERFAHREN ZUR HERSTELLUNG VON D,L-ASPARAGINSÄURE AUS AMMONIUMSALZEN DER MALEINSÄURE**
PROCESS FOR PREPARING D,L-ASPARTIC ACID FROM AMMONIUM SALTS OF THE MALEIC ACID
PROCEDE DE PREPARATION D'ACIDE ASPARTIQUE D,L A PARTIR DE SELS D'AMMONIUM DE L'ACIDE MALEIQUE

(30) Priorität: 24.09.1994 DE 4434172
(43) Veröffentlichungstag der Anmeldung: 14.05.1997
(73) Patentinhaber: BASF AKTIENGESELLSCHAFT, 67056 Ludwigshafen (DE)
(72) Erfinder: KRATZ, Detlef, D-69121 Heidelberg (DE); WITZEL, Tom, D-67069 Ludwigshafen (DE); BÄZNER, Rudolf, D-68259 Mannheim (DE); KRONER, Matthias, D-67304 Eisenberg (DE); PRESSLER, Uwe, D-67165 Waldsee (DE)
(86) Internationale Anmeldenummer: EP9503600
(87) Internationale Veröffentlichungsnummer: WO9609277

(56) Entgegenhaltungen:
- EP-A- 0 376 123
- DE-A- 2 029 502
- GB-A- 816 596

## Beschreibung

Die Erfindung betrifft ein Verfahren zur Herstellung von D,L-Asparaginsäure durch Erhitzen wäßriger Lösungen von Ammoniumsalzen der Maleinsäure auf höhere Temperaturen unter Druck, Ansäuern der Reaktionslösung unter Freisetzung von D,L-Asparaginsäure und Isolieren der D,L-Asparaginsäure.

Aus der DE-A-2 029 502 ist die Herstellung von D,L-Asparaginsäure durch Erhitzen von wäßrigen Lösungen des Diammoniumsalzes der Maleinsäure auf Temperaturen von 110 bis 145°C unter einem Druck von 2 bis 5 bar bekannt. Die wäßrige Lösung des Diammoniumsalzes der Maleinsäure wird durch Neutralisieren einer wäßrigen Maleinsäurelösung mit wäßrigem Ammoniak bei Temperaturen von max. 20°C hergestellt, wobei der pH-Wert der Ammoniumsalzlösung 7,5 beträgt. Die so erhältliche Reaktionslösung wird mit starken Mineralsäuren, wie Salzsäure, bis zu einem pH-Wert von 3 angesäuert, um die Asparaginsäure aus dem zunächst gebildeten Monoammoniumsalz in Freiheit zu setzen. Gemäß dem Beispiel in der Anmeldung beträgt die Ausbeute an D,L-Asparaginsäure nach dem Umkristallisieren aus Wasser 61 % der Theorie. Die Raum-Zeit-Ausbeuten sind jedoch unbefriedigend.

Nach dem aus der DD-A-126 075 bekannten Verfahren wird D,L-Asparaginsäure hergestellt, indem man auf bekannte Weise durch Neutralisation von Maleinsäureanhydrid mit wäßrigem Ammoniak das Diammoniumsalz der Maleinsäure herstellt, es unter Normaldruck bis zu einer Temperatur von 115°C eindampft, ein Carbonsäureamid als Katalysator zusetzt und die erhaltene wäßrige Lösung weiter unter Normaldruck bis zu einer Temperatur von 120 bis 130°C eindampft und das Reaktionsgemisch 16 Stunden bei dieser Temperatur rührt. Danach wird es auf 80°C abgekühlt, mit Salzsäure versetzt und zum Sieden unter Rückfluß erhitzt, um den Katalysator zu zerstören. Danach wird die überschüssige Salzsäure im Vakuum abdestilliert, der Rückstand in Wasser aufgenommen und die Asparaginsäure durch Einstellen der Lösung auf einen pH-Wert von 2,8 ausgefällt. Die Ausbeute an D,L-Asparaginsäure beträgt nach dem Umkristallisieren 63 %.

Aus der JP-A-25 133/65 ist die Herstellung von D,L-Asparaginsäure durch Umsetzung von Maleinsäure mit Ammoniak in Gegenwart von Ammoniumverbindungen wie Ammoniumchlorid oder Ammoniumacetat bekannt. Die Asparaginsäure wird aus ihrem Ammoniumsalz durch Zugabe von Maleinsäure zu der Reaktionslösung freigesetzt. Dabei stellt man einen pH-Wert von 2,5 bis 3 ein. Die dabei in kristallisierter Form anfallende D,L-Asparaginsäure wird abgesaugt und die Mutterlauge, die Ammoniummaleinat enthält, wieder eingesetzt.

Aus der US-A 4,560,653 ist die Herstellung von L-Asparaginsäure durch enzymatische Addition von Ammoniak an Fumarsäure bekannt. Aus der erhaltenen L-Ammoniumaspartat-Lösung wird L-Asparaginsäure durch Zugabe von Maleinsäure ausgefällt. Nach der Abtrennung von L-Asparaginsäure wird die verbleibende Mutterlauge einem Isomerisierungsschritt unterworfen, bei dem Maleinsäure in Fumarsäure umgewandelt wird, anschließend gereinigt und dann der enzymatischen Addition erneut zugeführt.

Der vorliegenden Erfindung liegt die Aufgabe zugrunde, ein Verfahren zur Herstellung von D,L-Asparaginsäure zur Verfügung zu stellen, das höhere Ausbeuten an D,L-Asparaginsäure liefert, als die bekannten Verfahren.

Die Aufgabe wird erfindungsgemäß gelöst mit einem Verfahren zur Herstellung D,L-Asparaginsäure durch Erhitzen wäßriger Lösungen von Ammoniumsalzen der Asparaginsäure auf höhere Temperaturen unter Druck, Ansäuern der Reaktionslösung unter Freisetzung von D,L-Asparaginsäure und Isolieren der D,L-Asparaginsäure, wenn man Maleinsäure und Ammoniak in einem Molverhältnis von 1:2,1 bis 1:50 in wäßriger Lösung bei Temperaturen von 60°C bis 250°C und Drücken von mindestens 1 bar umsetzt, wobei der Druck während der Umsetzung so reguliert wird, daß das Reaktionsgemisch fast vollständig in flüssiger Phase vorliegt.

Im Gegensatz zu den Verfahren des Standes der Technik wird bei dem erfindungsgemäßen Verfahren die Reaktion von Ammoniumsalzen der Maleinsäure in wäßriger Lösung so durchgeführt, daß die Reaktionslösung unter den Reaktionsbedingungen annähernd quantitativ in kondensierter Phase vorliegt. Dadurch wird gewährleistet, daß in der Lösung eine maximale Konzentration an Ammoniak zur Reaktion mit Maleinsäure zur Verfügung steht.

Das erfindungsgemäße Verfahren kann kontinuierlich oder diskontinuierlich durchgeführt werden. Die Lösungen von Ammoniumsalzen der Maleinsäure werden in bekannter Weise hergestellt. Man kann entweder von Maleinsäure oder von Maleinsäureanhydrid ausgehen, das in Wasser bekanntlich zu Maleinsäure hydrolysiert. Maleinsäure wird vorzugsweise in wäßriger Lösung mit Ammoniak neutralisiert. Hierbei kann man so vorgehen, daß man eine Lösung von Ammoniak in Wasser, vorzugsweise verwendet man konzentrierten Ammoniak, mit einer wäßrigen Lösung von Maleinsäure vereinigt. Es ist jedoch auch möglich, gasförmigen Ammoniak in eine wäßrige Lösung von Maleinsäure einzuleiten. Maleinsäure und Ammoniak werden bei dem erfindungsgemäßen Verfahren in einem Molverhältnis von 1:2,1 bis 1:50, vorzugsweise 1:2,1 bis 1:10 eingesetzt. Bezogen auf den Maleinsäuregehalt beträgt die Konzentration der wäßrigen Lösungen an Ammoniummaleinat 5 bis 40, vorzugsweise 10 bis 30 Gew.-%. Die wäßrige Lösung der Ammoniumsalze der Maleinsäure enthält vorzugsweise einen Überschuß an Ammoniak. Besonders bevorzugt setzt man solche wäßrigen Lösungen ein, bei denen das Molverhältnis von Maleinsäure : Ammoniak 1:3 bis 1:7 beträgt. Der pH-Wert der wäßrigen Lösungen von Ammoniummaleinat liegt z.B. in dem Bereich von 7,5 bis 11,5, vorzugsweise 8,5 bis 11.

Die wäßrigen Lösungen der Ammoniumsalze der Maleinsäure werden in einem Druckgefäß unter einem Druck von mindestens 1 bar umgesetzt. Als Druckgefäße kommen beispielsweise Autoklaven oder Rohrreaktoren in Betracht. Man kann auch Autoklaven und Rohrreaktoren hintereinander schalten oder die Umsetzung auch kontinuierlich in einer Kaskade aus 2 oder 3 bis 4 Autoklaven, die mit einem Rührer ausgestattet sind, durchführen. Die Reaktion kann auch so durchgeführt werden, daß im Laufe der Reaktion Ammoniak nachdosiert wird. Die Druckgefäße werden erfindungsgemäß vollständig mit der wäßrigen Lösung der Ammoniumsalze der Maleinsäure gefüllt, so daß über der Flüssigkeit praktisch kein Gasraum vorhanden ist. Die wäßrigen Lösungen werden in der Praxis in das Druckgefäß gepumpt, bis sich der Druck eingestellt hat, unter dem die Reaktion ablaufen soll. Dieser Druck beträgt mindestens 1 bar und liegt üblicherweise in dem Bereich von 2 bis 100 bar. Man kann jedoch auch bei höheren Drücken arbeiten, jedoch bedeutet dies für die Ausbeute und den Umsatz an D,L-Asparaginsäure praktisch keinen Vorteil. Das Arbeiten bei sehr hohen Drücken erfordert eine entsprechende Auslegung der Apparaturen, so daß man das erfindungsgemäße Verfahren aus Kostengründen eher bei niedrigeren Drücken durchführt, z.B. in dem Druckbereich von 2 bis 90, vorzugsweise 5 bis 80 und insbesondere 10 bis 40 bar. Die Umsetzung kann auch dadurch praktisch in vollständig flüssiger Phase durchgeführt werden, in dem man bei einer vorgegebenen Lösungsmenge den Druck durch Aufpressen eines Inertgases, beispielsweise Stickstoff, reguliert. Die Lösung kann gegebenenfalls inerte Lösemittel wie Ether, z.B. Dioxan, Tetrahydrofuran, Diisopropylether, Methyl-tert-butylether oder Kohlenwasserstoffe wie Toluol, Xylol, Octan oder Decan enthalten. Außerdem ist es möglich, N-Methylpyrrolidon als Lösemittel zu verwenden. Falls die wäßrige Lösung der Ammoniumsalze der Maleinsäure ein von Wasser verschiedenes inertes Lösemittel enthält, betragen die Mengen an inertem Lösemittel z.B. l bis 50 Gew.-%, bezogen auf die gesamte Menge an Lösemittel.

Durch Erhitzen der wäßrigen Lösungen von Ammoniumsalzen der Maleinsäure auf Temperaturen in dem Bereich von beispielsweise 60 bis 250, vorzugsweise 100 bis 200°C erhält man D,L-Asparaginsäure. Die Umsetzung kann gegebenenfalls in Gegenwart eines heterogenen Katalysators vorgenommen werden. Geeignete, heterogene Katalysatoren, die sich weder in der Ausgangslösung der Ammoniumsalze noch in der Reaktionsmischung lösen, sind beispielsweise Aluminiumoxid, Siliciumdioxid, Titandioxid, Magnesiumoxid, Zinkoxid sowie Mischoxide wie Spinellarten, Titan-Wolframoxide, Zinnaluminium-oxide oder solche Katalysatoren, die z.B. mit Übergangsmetallen der Nebengruppen 1 bis 8 des Periodischen Systems dotiert sind und Zirkondioxid. Diese Trägermaterialien können gegebenenfalls mit alkalischen Komponenten wie Alkalimetall- oder Erdalkalimetalloxiden oder sauren Zusätzen, z.B. Phosphorsäure, dotiert sein. Außerdem sind Ionenaustauscher mit sauren oder basischen Gruppen als Katalysator geeignet. Bei den Ionenaustauschern handelt es sich um vernetzte Polymerisate, die in den erfindungsgemäß zum Einsatz gelangenden wäßrigen Reaktionsmedien unlöslich sind.

Die Verweilzeit der Reaktionslösung im Druckreaktor beträgt beispielsweise 1 bis 300, vorzugsweise 15 bis 120 Minuten. Die Verweilzeit hängt dabei im wesentlichen von der jeweils gewählten Reaktionstemperatur ab. Bei höheren Temperaturen benötigt man geringere Verweilzeiten. Die aus dem Reaktor kommende Reaktionslösung wird entspannt und kondensiert. Sie enthält bis zu 85 % Asparaginsäure in Form des Ammoniumsalzes sowie Nebenkomponenten wie Fumarsäure, Iminodibernsteinsäure, Äpfelsäure und auch geringe Mengen an Amiden wie Asparagin. Wenn mit einem Überschuß an Ammoniak, bezogen auf Maleinsäure gearbeitet wurde, wird vor einer Freisetzung der D,L-Asparaginsäure mit Säuren, vorzugsweise Maleinsäure, überschüssiges Ammoniak aus der wäßrigen Reaktionslösung abdestilliert. Die Destillation des Ammoniaks kann drucklos, unter erhöhtem Druck oder auch unter vermindertem Druck, z.B. in dem Bereich von 1 mbar bis 50 bar durchgeführt werden. Das als Leichtsieder erhaltene Gemisch aus Wasser und Ammoniak kann erneut zur Herstellung von D,L-Asparaginsäure verwendet werden, indem man damit Maleinsäure mit Ammoniak in einem solchen Molverhältnis neutralisiert, wie es erfindungsgemäß erforderlich ist. Beim Entfernen von überschüssigem Ammoniak wird die Reaktionslösung meistens aufkonzentriert. Der Feststoffgehalt der Reaktionslösung beträgt dann etwa 10 bis 50, vorzugsweise 20 bis 40 Gew.-%. Nach dem Entfernen von überschüssigem Ammoniak hat die Reaktionslösung einen pH-Wert von beispielsweise 4,7 bis 6,5, vorzugsweise 5,2 bis 6,0. Hauptbestandteil der wäßrigen Lösung mit mindestens 15 Gew.-% ist das Ammoniumsalz der Asparaginsäure. Die Reaktionsmischung enthält außerdem Ammoniumsalze der anderen obengenannten Säuren, die als Nebenprodukt bei der Umsetzung entstehen.

In der bevorzugten Ausführungsform des erfindungsgemäßen Verfahrens führt man die Umsetzung der Ammoniumsalze der Maleinsäure bei Temperaturen von 120 bis 180°C durch, entfernt überschüssigen Ammoniak aus der Reaktionslösung, säuert dann die Reaktionslösung durch Zugabe von Maleinsäure oder Maleinsäureanhydrid an, trennt die D,L-Asparaginsäure aus der so erhältlichen Mutterlauge ab und führt die Mutterlauge und den überschüssigen Ammoniak zur Herstellung von Ammoniumsalzen der Maleinsäure in das Verfahren zur Herstellung von D,L-Asparaginsäure zurück.

Das Ansäuern der Reaktionslösung kann prinzipiell auch mit Mineralsäuren, wie Schwefelsäure oder Salzsäure oder anderen Säuren wie para-Toluolsulfonsäure erfolgen, jedoch kann man die dann entstehenden Ammoniumsalze nicht in den Prozeß zurückführen. Aus diesem Grunde ist das Ansäuern der Reaktionslösung unter Freisetzung von D,L-Asparaginsäure mit Maleinsäure oder Maleinsäureanhydrid bevorzugt.

Verwendet man zum Ansäuern der Reaktionslösung Schwefelsäure oder Salzsäure, so wird in literaturbekannterweise bis zu einem pH-Wert von 2,5 bis 3 angesäuert und Asparaginsäure nach konventionellen Methoden wie Filtrieren oder Zentrifugieren abgetrennt. Bezogen auf den Gehalt an Asparaginsäure in der Reaktionslösung kann man so etwa 90 % der Asparaginsäure isolieren. Fumarsäure und Iminodibernsteinsäure bleiben unter diesen Bedingungen in Lösung. Der Nachteil dieser Isolierungsmethode liegt darin, daß damit ein Verlust von mindestens 10 % der Asparaginsäure verbunden ist, weil die in der Lösung noch verbliebene Fumarsäure oder Iminodibernsteinsäure nicht verwertet werden kann und daß ein anorganisches Ammoniumsalz in äquimolaren Mengen anfällt.

Beim Ansäuern der Reaktionslösung mit Maleinsäure, die entweder in fester Form oder als wäßrige Lösung, die beispielsweise auch durch Hydrolyse von Maleinsäureanhydrid hergestellt werden kann, wird der pH-Wert der Lösung auf etwa 3 eingestellt. Dadurch kann man ebenfalls ca. 90 % der Asparaginsäure aus der Reaktionslösung ausfällen. Man muß jedoch, bezogen auf die ausgefällte D,L-Asparaginsäure, gegebenenfalls einen stöchiometrischen Überschuß an Maleinsäure der Reaktionslösung zufügen. Je nach Fällungsbedingungen stellt man einen pH-Wert von 3,5 bis 5 ein, weil dann die zugegebene Menge an Maleinsäure der erhaltenen Menge an Asparaginsäure entspricht. Diese Methode der Fällung von D,L-Asparaginsäure aus der Reaktionslösung ist besonders dann geeignet, wenn die nach der Isolierung von Asparaginsäure erhaltene Mutterlauge erneut zur Herstellung von D,L-Asparaginsäure in den Prozeß zurückgeführt wird. Das im Kreislauf vorhandene Volumen bleibt somit konstant.

Nach dem Abtrennen und dem Waschen der D,L-Asparaginsäure fällt eine Mutterlauge an, die hauptsächlich noch Asparaginsäure, Maleinsäure, Fumarsäure und Iminodibernsteinsäure enthält. Das Waschwasser enthält ebenfalls die vorstehend genannten Säuren. Die Mutterlauge und das Waschwasser können nach Reaktion mit Ammoniak zur Herstellung von D,L-Asparaginsäure erfindungsgemäß eingesetzt werden, wobei man die genannten Säuren mit Ammoniak vorzugsweise in einem Molverhältnis von 1:2,1 bis 1:10 einsetzt. Überraschenderweise entspricht die bei einem erneuten Umsatz nach dem erfindungsgemäßen Verfahren erhaltene Reaktionslösung bezüglich der organischen Bestandteile der direkten Umsetzung von Ammoniumsalzen der Maleinsäure gemäß dem erfindungsgemäßen Verfahren. Durch die Neutralisation der Reaktionslösung mit Maleinsäure und die Rückführung der Mutterlauge in den Prozeß ist es also möglich, D,L-Asparaginsäure ohne den Anfall anorganischer Salze zu erhalten und gleichzeitig die neben D,L-Asparaginsäure entstehenden Nebenprodukte wie Fumarsäure, Äpfelsäure und Iminodibernsteinsäure quantitativ zur Herstellung von D,L-Asparaginsäure zu verwerten. Durch mehrmalige Wiederholung der Aufarbeitungsschritte und Rückführen der Mutterlauge und von überschüssigem Ammoniak und erneuter Fällung mit Maleinsäure wird eine Ausbeute von mehr als 98 % D,L-Asparaginsäure, bezogen auf Maleinsäure, erreicht.

Besonders bevorzugt ist die kontinuierliche Durchführung des erfindungsgemäßen Verfahrens, bei dem beispielsweise eine zurückgeführte Mutterlauge mit zurückgeführtem Ammoniak und Zugabe von frischem Ammoniak und gegebenenfalls frischer Maleinsäure eine wäßrige Lösung von Ammoniumsalzen der Maleinsäure in vorgesehenem Verhältnis hergestellt und in einem Reaktor bei Temperaturen von 60 bis 250°C und Drücken von mindestens 1 bar umgesetzt wird, wobei der Druck während der Umsetzung so reguliert wird, daß das Reaktionsgemisch fast vollständig in flüssiger Phase vorliegt. Die dabei entstehende Ammoniumaspartatlösung wird von überschüssigem Ammoniak befreit und dabei gleichzeitig aufkonzentriert. Der erhaltene wäßrige Ammoniak wird zurückgeführt. Man kann jedoch auch den wäßrigen Ammoniak auftrennen, so daß man konzentrierten Ammoniak und Wasser erhält. Beide Stoffe können zurückgeführt werden, wobei Wasser zur Herstellung einer wäßrigen Lösung von Maleinsäure eingesetzt oder zum Waschen von Asparaginsäure verwendet wird. Nach der Entfernung von überschüssigem Ammoniak gibt man eine wäßrige Lösung von Maleinsäure zur Reaktionslösung, wobei man einen pH-Wert in dem Bereich von 3,5 bis 5 einstellt, so daß D,L-Asparaginsäure ausfällt. Die ausgefallene D,L-Asparaginsäure wird abgetrennt und mit Wasser gewaschen. Die Mutterlauge und das Waschwasser werden nach Zugabe von Ammoniak in den Prozeß zurückgeführt. Die D,L-Asparaginsäure kann gegebenenfalls umkristallisiert werden.

D,L-Asparaginsäure kann beispielsweise thermisch durch Erhitzen auf Temperaturen oberhalb von 190°C oder in Gegenwart von Säuren wie Phosphorsäure oder von sauren Ammoniumsalzen wie Ammoniumhydrogensulfat bei Temperaturen von z.B. 170 bis 230°C zu Polyaspartimiden bzw. zu Mischungen aus Polyaspartimiden und Polyasparaginsäure kondensiert werden. Die Polykondensate werden z.B. in Mengen von 1 bis 10 Gew.-% als Waschmitteladditiv, bezogen auf die Formulierung, verwendet.

Die Prozentangaben in den Beispielen bedeuten Gewichtsprozent. Die in den Beispielen angegebenen Zusammensetzungen der Reaktionslösungen wurden gaschromatographisch ermittelt (Flächenprozent).

### Beispiel 1

Man stellt zunächst eine Ammoniummaleinatlösung durch Mischen von 120 g Maleinsäure, 37 g Ammoniak und 243 g Wasser her. Daraus berechnet sich ein Gehalt an Maleinsäure in der Lösung von 30 %. Das molare Verhältnis von Maleinsäure:Ammoniak beträgt 1:2,1, der pH-Wert der Ammoniummaleinatlösung 8,5. Die Lösung wird kontinuierlich mit einer Förderleistung von 133 ml/h in einen Rohrreaktor gepumpt, der auf eine Temperatur von 160°C temperiert ist, eine Länge von 30 m, einen Durchmesser von 2 mm und ein Volumen von 100 ml hat. Der Druck in dem Rohrreaktor wird mit Hilfe eines Druckhaltesystems so reguliert, daß das Reaktionsgemisch praktisch vollständig in flüssiger Phase vorliegt. Der Druck beträgt 80 bar. Der Reaktoraustrag wird kondensiert und analysiert. Der Umsatz, bezogen auf Maleinsäure, beträgt 99,9 %. Das Reaktionsgemisch hatte folgende Zusammensetzung:

| | |
|---|---|
| 0,1 % | Maleinsäure |
| 4 % | Fumarsäure |
| 79 % | D,L-Asparaginsäure |
| 15 % | Iminodibernsteinsäure |
| 0,2 % | Äpfelsäure |
| 0,6 % | Asparagin und |
| 1,1 % | nicht identifizierte Verbindungen. |

Das Reaktionsgemisch wird durch Zugabe von Maleinsäure auf einen pH-Wert von 3,8 eingestellt. Dabei kristallisiert D,L-Asparaginsäure aus.

### Beispiel 2

Man stellt zunächst eine Mischung aus

| | |
|---|---|
| 69 % | Maleinsäure |
| 9 % | Fumarsäure |
| 9 % | D,L-Asparaginsäure und |
| 13 % | Iminodibernsteinsäure |

her und bereitet daraus eine 20 gew.-%ige wäßrige Lösung, die dann durch Zugabe von Ammoniak auf einen pH-Wert von 8,5 eingestellt wird. Diese Lösung wird dann durch den in Beispiel 1 angegebenen Reaktor unter den dort ebenfalls beschriebenen Bedingungen durchgesetzt. Die Zusammensetzung des Reaktionsgemisches entspricht nahezu der Zusammensetzung des Reaktionsgemisches aus Beispiel 1, und zwar enthält die Reaktionslösung

| | |
|---|---|
| 0,1 % | Maleinsäure |
| 4,5 % | Fumarsäure |
| 77 % | D,L-Asparaginsäure |
| 15 % | Iminodibernsteinsäure und |
| 3,4 % | nicht identifizierte Bestandteile. |

### Beispiel 3

Man stellt eine 20%ige wäßrige Lösung durch Mischen von 1 Mol Maleinsäure, Wasser und 2,1 Mol Ammoniak her. Die Lösung wird anschließend kontinuierlich mit einer Förderleistung von 24 ml/h durch ein Rohr gepumpt, das eine Länge von 30 cm, einen Durchmesser von 1,5 cm und ein Volumen von 60 ml hat. Das Rohr ist mit Glaskugeln gefüllt, die einen Durchmesser von 3 bis 5 mm haben. Das Rohr wird auf 180°C beheizt. Zur Einstellung des Drucks von 80 bar wird ein Überströmer verwendet. Während der Reaktion bei 180° unter einem Druck von 80 bar liegt das Reaktionsgemisch fast vollständig in flüssiger Phase vor. Der Reaktoraustrag wird kondensiert und analysiert. Der Umsatz beträgt 99,4 %. Die Zusammensetzung des Reaktionsgemischs beträgt

| | |
|---|---|
| 0,6 % | Maleinsäure |
| 7,5 % | Fumarsäure |
| 72 % | D,L-Asparaginsäure |
| 16 % | Iminodibernsteinsäure |
| 1,1 % | Äpfelsäure |
| 2,8 % | nicht identifizierte Bestandteile. |

Aus dem Reaktionsgemisch wird durch Zugabe von Maleinsäure zu einem pH-Wert von 4,7 D,L-Asparaginsäure in Form von Kristallen ausgefällt.

### Beispiel 4

Beispiel 1 wird mit der einzigen Ausnahme wiederholt, daß die Reaktortemperatur 180°C beträgt. Man erhält dann ein Reaktionsgemisch, das

| | |
|---|---|
| 0,1 % | Maleinsäure |
| 3 % | Fumarsäure |
| 83 % | D,L-Asparaginsäure |
| 7 % | Iminodibernsteinsäure |
| 0,5 % | Äpfelsäure |
| 0,5 % | L-Asparaginsäure und |
| 5,9 % | nicht identifizierte Bestandteile enthält. |

Durch Zugabe von Maleinsäure zur Reaktionslösung bis zu einem pH-Wert von 4,5 D,L-Asparaginsäure ausgefällt.

### Beispiel 5

Das Beispiel 1 wird mit der einzigen Ausnahme wiederholt, daß man die Reaktortemperatur auf 200°C erhöht. Das Reaktionsgemisch hat folgende Zusammensetzung:

| | |
|---|---|
| 0,5 % | Maleinsäure |
| 4 % | Fumarsäure |
| 78 % | D,L-Asparaginsäure |
| 6 % | Iminodibernsteinsäure |
| 1,4 % | Äpfelsäure |
| 2,5 % | L-Asparaginsäure und |
| 7,6 % | nicht identifizierte Bestandteile. |

Die Zugabe von Maleinsäure bis zu einem pH-Wert von 4,2 zur Reaktionslösung wird D,L-Asparaginsäure ausgefällt.

### Beispiel 6

Das Beispiel 3 wird mit dem einzigen Unterschied wiederholt, daß das Verhältnis von Maleinsäure : Ammoniak 1:3 beträgt. Die Reaktionslösung enthält dann

| | |
|---|---|
| 0,5 % | Maleinsäure |
| 4,5 % | Fumarsäure |
| 80 % | D,L-Asparaginsäure |
| 8 % | Iminodibernsteinsäure |
| 0,7 % | Äpfelsäure |
| 6,3 % | nicht identifizierte Bestandteile. |

Durch Zugabe von Maleinsäure bis zu einem pH-Wert von 4,7 zur Reaktionslösung wird D,L-Asparaginsäure ausgefällt.

### Beispiel 7

Das Beispiel 3 wird mit den Ausnahmen wiederholt, daß man jetzt ein Verhältnis von Maleinsäure : Ammoniak von 1:5 einstellt und die Reaktion bei 160°C durchführt. Das entstehende Reaktionsgemisch enthält dann

| | |
|---|---|
| 0,4 % | Maleinsäure |
| 4 % | Fumarsäure |
| 83 % | D,L-Asparaginsäure |
| 4 % | Iminodibernsteinsäure und |
| 8,6 % | nicht identifizierte Stoffe. |

### Beispiel 8

Beispiel 3 wird mit den Ausnahmen wiederholt, daß man anstelle der Glaskugeln jetzt 1,5 mm Stränge aus Aluminiumoxid als Katalysator verwendet und die Zulaufgeschwindigkeit auf 60 ml/h einstellt. Die aus dem Reaktor kommende Reaktionslösung hat folgende Zusammensetzung:

| | |
|---|---|
| 0,1 % | Maleinsäure |
| 4,4 % | Fumarsäure |
| 89 % | D,L-Asparaginsäure |
| 1 % | Iminodibernsteinsäure |
| 2,5 % | Äpfelsäure und |
| 3 % | nicht identifizierte Anteile. |

Durch Zugabe von Maleinsäure zur Reaktionslösung bis zu einem pH-Wert von 4,0 wird D,L-Asparaginsäure ausgefällt.

### Beispiel 9

3000 ml einer 20 %igen wäßrigen Lösung aus 600 g (5,17 Mol) Maleinsäure, die mit 264 g (15,51 Mol) Ammoniak neutralisiert ist, werden durch das in Beispiel 1 beschriebene und auf 160°C temperierte Reaktorrohr unter den übrigen dort angegebenen Bedingungen gepumpt. Das den Reaktor verlassende Reaktionsgemisch enthält

| | |
|---|---|
| 0,4 % | Maleinsäure |
| 5 % | Fumarsäure |
| 84 % | D,L-Asparaginsäure |
| 8 % | Iminodibernsteinsäure und |
| 2,6 % | nicht identifizierte Stoffe. |

Der pH-Wert des Zulaufs beträgt 10,5, der des kondensierten Reaktionsgemisches 9,8.

Man destilliert den überschüssigen Ammoniak aus der Reaktionslösung ab und gibt anschließend so viel Maleinsäure zu, daß der pH-Wert der Lösung 4,3 beträgt. Dabei fällt D,L-Asparaginsäure aus. Die Mutterlauge und der abdestillierte Ammoniak können erneut zusammen mit frischem Ammoniummaleinat zur Synthese von D,L-Asparaginsäure eingesetzt werden, vgl. Beispiele 10 bis 12.

### Beispiel 10

3000 ml des Reaktionsaustrags aus Beispiel 9 werden in eine Destillationsapparatur gefüllt. Bei einer Sumpftemperatur von 100°C destilliert man 1432 ml eines Gemischs aus Wasser und Ammoniak bei Normaldruck ab. Der pH-Wert des Sumpfes beträgt am Ende der Destillation 5,85. Der Destillationssumpf enthält

| | |
|---|---|
| 0,4 % | Maleinsäure |
| 7 % | Fumarsäure |
| 81 % | D,L-Asparaginsäure und |
| 9 % | Iminodibernsteinsäure. |

Der Rest besteht aus nicht identifizierten Substanzen.

### Beispiel 11

Zu dem Sumpf aus Beispiel 10 gibt man portionsweise bei 20°C insgesamt 348 g (3 Mol) Maleinsäure hinzu. Der pH-Wert der Lösung sinkt dabei kurzzeitig bis auf ca. 4, steigt aber an, sobald D,L-Asparaginsäure auszufallen beginnt. Die Mischung wird 8 Stunden bei 20°C gerührt, wobei der pH-Wert zuletzt 4,75 beträgt. Der Niederschlag wird danach abgesaugt und mit 1000 ml Wasser gewaschen. Nach dem Trocknen erhält man 427 g (3,21 Mol) D,L-Asparaginsäure entsprechend einer Ausbeute von 62 %, bezogen auf die im Beispiel 9 eingesetzte Maleinsäuremenge.

### Beispiel 12

Die Mutterlauge und das Waschwasser aus Beispiel 11 werden vereinigt. Man fügt dann soviel Wasser und Ammoniak hinzu, daß man 2780 ml einer 20%igen wäßrigen Lösung mit einem pH-Wert von 10,5 erhält. Diese Lösung wird, wie in Beispiel 9 beschrieben, zur Reaktion gebracht und anschließend analog Beispiel 10 aufkonzentriert. Es verbleiben 1453 ml eines Sumpfes. Die Fällung von D,L-Asparaginsäure erfolgt dann, wie in Beispiel 11 beschrieben, durch Zugabe von 323 g (2,78 Mol) Maleinsäure. Nach dem Trocknen erhält man 382 g (2,87 Mol) D,L-Asparaginsäure entsprechend einer Ausbeute von 95,7 %, bezogen auf die zugegebene Menge Maleinsäure aus Beispiel 11.

### Beispiel 13

Das Beispiel 3 wird mit den Ausnahmen wiederholt, daß der Reaktor keine Glaskugeln enthält und der Druck 15 bar beträgt. Die Zusammensetzung des Reaktionsgemisches entspricht praktisch der in Beispiel 3 angegebenen Zusammensetzung des Reaktionsgemisches.

## Patentansprüche

1. Verfahren zur Herstellung von D,L-Asparaginsäure durch Erhitzen wäßriger Lösungen von Ammoniumsalzen der Maleinsäure auf höhere Temperaturen unter Druck, Ansäuern der Reaktionslösung unter Freisetzung von D,L-Asparaginsäure und Isolieren der D,L-Asparaginsäure, dadurch gekennzeichnet, daß man Maleinsäure und Ammoniak in einem Molverhältnis von 1:2,1 bis 1:50 in wäßriger Lösung bei Temperaturen von 60 bis 250°C und Drücken von mindestens 1 bar umsetzt, wobei der Druck während der Umsetzung so reguliert wird, daß das Reaktionsgemisch fast vollständig in flüssiger Phase vorliegt.

2. Verfahren nach Anspruch 1, dadurch gekennzeichnet, daß man Maleinsäure und Ammoniak in einem Molverhältnis von 1:3 bis 1:7 einsetzt.

3. Verfahren nach Anspruch 1 oder 2, dadurch gekennzeichnet, daß man die Umsetzung in einem Rohrreaktor durchführt.

4. Verfahren nach einem der Ansprüche 1 bis 3, dadurch gekennzeichnet, daß die Konzentration an Ammoniummaleinat, bezogen auf Maleinsäure, in den wäßrigen Lösungen 5 bis 40 Gew.-% beträgt.

5. Verfahren nach einem der Ansprüche 1 bis 4, dadurch gekennzeichnet, daß man die Umsetzung bei Temperaturen von 120 bis 180°C und Drücken von 2 bis 40 bar durchführt.

6. Verfahren nach einem der Ansprüche 1 bis 5, dadurch gekennzeichnet, daß man überschüssigen Ammoniak aus der Reaktionslösung entfernt, die Reaktionslösung dann durch Zugabe von Maleinsäure oder Maleinsäureanhydrid ansäuert, D,L-Asparaginsäure aus der so erhältlichen Mutterlauge abtrennt und die Mutterlauge und den überschüssigen Ammoniak zur Herstellung von Ammoniumsalzen der Maleinsäure in das Verfahren zur Herstellung von D,L-Asparaginsäure zurückführt.

7. Verfahren nach einem der Ansprüche 1 bis 6, dadurch gekennzeichnet, daß es kontinuierlich durchgeführt wird.

## Claims

1. A process for preparing D,L-aspartic acid by heating aqueous solutions of ammonium salts of maleic acid at elevated temperatures under pressure, acidifying the reaction solution to liberate D,L-aspartic acid and isolating the D,L-aspartic acid, wherein maleic acid and ammonia are reacted in a molar ratio of from 1:2,1 to 1:50 in aqueous solution at from 60 to 250°C under pressures of at least 1 bar, the pressure being controlled during the reaction in such a way that the reaction mixture is almost entirely in the liquid phase.

2. A process as claimed in claim 1, wherein maleic acid and ammonia are employed in a molar ratio of from 1:3 to 1:7.

3. A process as claimed in claim 1 or 2, wherein the reaction is carried out in a tubular reactor.

4. A process as claimed in any of claims 1 to 3, wherein the concentration of ammonium maleate based on maleic acid in the aqueous solutions is from 5 to 40 % by weight.

5. A process as claimed in any of claims 1 to 4, wherein the reaction is carried out at from 120 to 180°C under from 2 to 40 bar.

6. A process as claimed in any of claims 1 to 5, wherein excess ammonia is removed from the reaction solution, the reaction solution is then acidified by adding maleic acid or maleic anhydride, D,L-aspartic acid is separated from the mother liquor obtainable in this way, and the mother liquor and the excess ammonia are returned, for preparing ammonium salts of maleic acid, to the process for preparing D,L-aspartic acid.

7. A process as claimed in any of claims 1 to 6, which is carried out continuously.

## Revendications

1. Procédé de préparation d'acide D,L-aspartique par chauffage à hautes températures et sous pression de solutions aqueuses de sels d'ammonium d'acide maléique, acidification de la solution réactionnelle pour la libération de l'acide D,L-aspartique et isolement de l'acide D,L-aspartique, caractérisé en ce que l'on fait réagir de l'acide maléique et de l'ammoniaque dans un rapport en moles de 1:2,1 à 1:50 en solution aqueuse à des températures de 60-250°C et sous des pressions d'au moins 1 bar, la pression pendant la réaction étant régulée de sorte que le mélange réactionnel soit presque entièrement en phase liquide.

2. Procédé selon la revendication 1, caractérisé en ce que l'on introduit l'acide maléique et l'ammoniaque dans un rapport en moles de 1:3 à 1:7.

3. Procédé selon la revendication 1 ou 2, caractérisé en ce que l'on mène la réaction dans un réacteur tubulaire.

4. Procédé selon l'une quelconque des revendications 1 à 3, caractérisé en ce que la concentration en maléate d'ammonium dans les solutions aqueuses, s'élève à 5-40% en poids, par rapport à l'acide maléique.

5. Procédé selon l'une quelconque des revendications 1 à 4, caractérisé en ce que l'on mène la réaction à des températures de 120-180°C et sous des pressions de 2-40 bar.

6. Procédé selon l'une quelconque des revendications 1 à 5, caractérisé en ce que l'on élimine l'ammoniaque en excès de la solution réactionnelle, on acidifie ensuite la solution réactionnelle par addition d'acide maléique ou d'anhydride maléique, on sépare l'acide D,L-aspartique de la solution mère ainsi obtenue et l'on réintroduit la solution mère et l'ammoniaque en excès dans le procédé de préparation d'acide D,L-aspartique pour la préparation de sels d'ammonium de l'acide maléique.

7. Procédé selon l'une quelconque des revendications 1 à 6, caractérisé en ce qu'on le mène de façon continue.
